# EUROPEAN PATENT APPLICATION

(11) **EP 2 889 022 A1**
(43) Date of publication of application: **01.07.2015**
(21) Application number: 13006059.3
(22) Date of filing: 30.12.2013
(51) Int. Cl.: A61F 9/06, A61F 9/02

(54) **Protection mask**

(71) Applicant: Wang, Ming-Cheng, Yung Kang City, Tainan (TW)
(72) Inventor: Wang, Ming-Cheng, Yung Kang City, Tainan (TW)
(74) Representative: Zeitler Volpert Kandlbinder Patent- und Rechtsanwälte Partnerschaft mbB

(57) **Abstract**

A protection mask includes a frame 1 having two slits 10 in two ends thereof and a tongue 11 is located in each slit 10. Two temples 12 extend backward from the two ends of the frame 1. A connection 2 rod is located at the middle portion of the frame 1. A connector 2 is connected to the connection rod 13 and has a C-clip 20 which has a notch 21. A protrusion 22 extends from the connector 2 and has a groove 23 defined in the outside thereof. A nose pad 3 is connected to the connection rod 13 and has a snapping portion 30 which is engaged with the notch 21 of the connector 2. The snapping portion 30 has a snapping slot 31. A protection member 4 has an insertion slot 40 with which the protrusion 22 of the connector 2 is engaged. Two connection slots 41 are respectively defined in two ends of the protection member 4. The two tongues 11 are engaged with the two connection slots 41.

## Description

### BACKGROUND OF THE INVENTION

### 1. Fields of the invention

The present invention relates to a protection mask, and more particularly, to a protection mask with adjustable nose pad so as to well protect the wearer's face and eyes.

### 2. Descriptions of Related Art

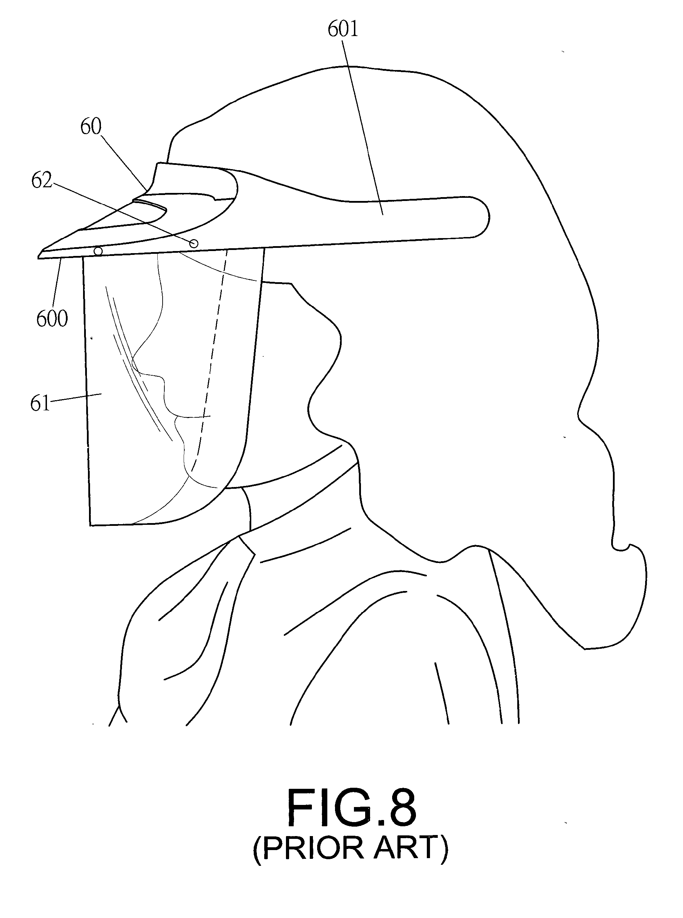

The conventional sunglasses are used to protect the eyes from the ultra-violet rays and cannot protect the wearer's face from being directly accessed by ultra-violet rays and dusts. Besides, the people work in the kitchens need a mask to protect their faces and eyes from splashed oil. The medical care persons also need the protection masks to avoid from the patients' secretions and saliva. Fig. 8 shows the conventional protection mask and comprises a frame 60 and a protection member 61. The frame 60 has a visor 600 and two extensions 601 extend from the visor 600 so that the wearer's head is positioned between the two extensions 601. The protection member 61 is connected to the underside of the visor 600 by multiple fixing members 62. However, the weight of the protection member 61 usually drag the frame 60 downward so that the wearer has to adjust the mask frequently. Besides, the protection member 61 is fixed to the frame 60 so that the protection member 61 cannot be adjustable according to the wearer's face. The protection member 61 cannot be folded so that the conventional protection mask occupy a certain space which is not convenient for storage.

The present invention intends to provide a protection mask wherein the protection member is foldable and the nose pad is adjustable.

### SUMMARY OF THE INVENTION

The present invention relates to a protection mask and comprises a frame has two slits defined in two ends thereof. Each slit has a tongue located therein. Two temples extend backward from the two ends of the frame. A connection rod is located at the middle portion of the frame. A connector is connected to the connection rod of the frame and has a C-clip on one end thereof. The C-clip has a notch in the middle portion thereof. A protrusion extends from the connector and has a groove defined in an outside thereof. A nose pad is connected to the connection rod of the frame and has a snapping portion which is engaged with the notch of the connector. The snapping portion has a snapping slot. A protection member has an insertion slot defined through the middle portion thereof as so to be connected in front of the frame. The protrusion of the connector is engaged with the insertion slot. Two connection slots are respectively defined in two ends of the protection member. The two tongues are engaged with the two connection slots.

Preferably, the connection rod of the frame has at least one tooth. Multiple engaging slots are defined in the inside of the snapping slot of the nose pad. The at least one tooth is engaged with one of the engaging slots of the snapping slot of the nose pad so that the nose pad is adjustable and positioned.

Preferably, the protection member is a transparent plate.

Preferably, the protection member is a translucent and colored plate which stops ultra-violet rays from penetrating through the protection member.

The present invention is easily assembled and the protection member is foldable relative to the frame to save space required so that the present invention is easily carried.

The folded protection mask of the present invention is compact in size so as to save the packing material.

The nose pad of the protection mask of the present invention is adjustable according to the wearers of different requirements, such that the protection mask of the present invention is well supported and does not shake and slip.

The protection mask of the present invention prevents splash oil and smoke from accessing the wearer's face and eyes when working in the kitchen.

The protection mask of the present invention protects the wearer's face from being directly accessed by ultra-violet rays and dusts when in outdoor exercises.

The protection mask of the present invention protects the medical care persons from being touched by the patients' secretions and saliva.

The protection member of the protection mask of the present invention is easily separated from the frame and replaced with a new protection member.

The present invention will become more obvious from the following description when taken in connection with the accompanying drawings which show, for purposes of illustration only, a preferred embodiment in accordance with the present invention.

### BRIEF DESCRIPTION OF THE DRAWINGS

Fig. 1 is an exploded view of the protection mask of the present invention;
Fig. 2 is an enlarged view to show the circled A in Fig. 1 of the protection mask of the present invention;
Fig. 3 is a perspective view to show the protection mask of the present invention;
Fig. 4 is a cross sectional view of the protection mask of the present invention;
Fig. 5 is an enlarged view to show the circled B in Fig. 4 of the protection mask of the present invention;
Fig. 6 shows that a wear wears the protection mask of the present invention;
Fig. 7 shows that the folded protection mask of the present invention is received in a bag, and
Fig. 8 shows that a wear wears the conventional protection mask.

### DETAILED DESCRIPTION OF THE PREFERRED EMBODIMENT

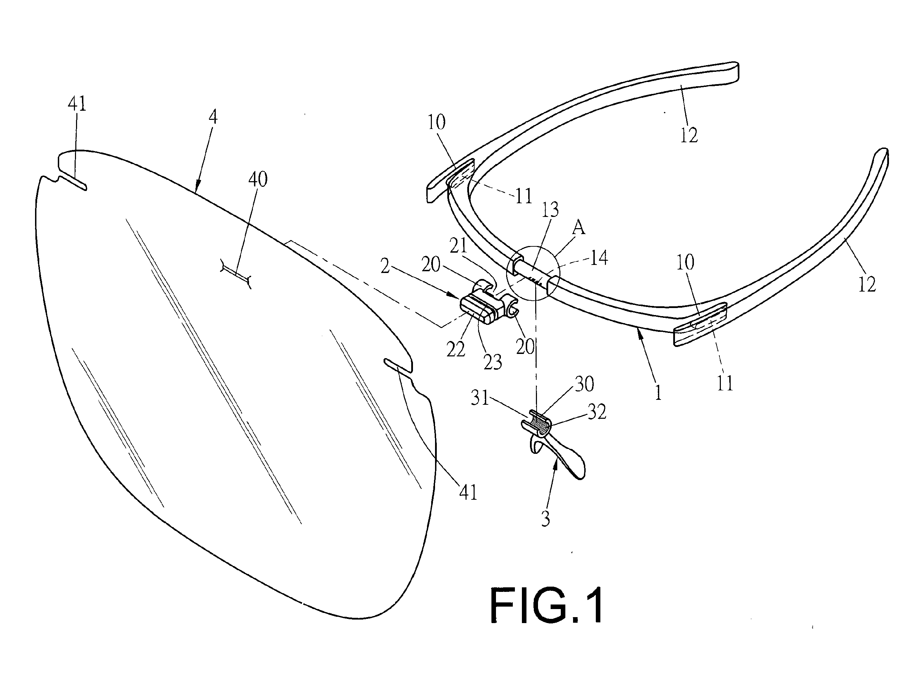

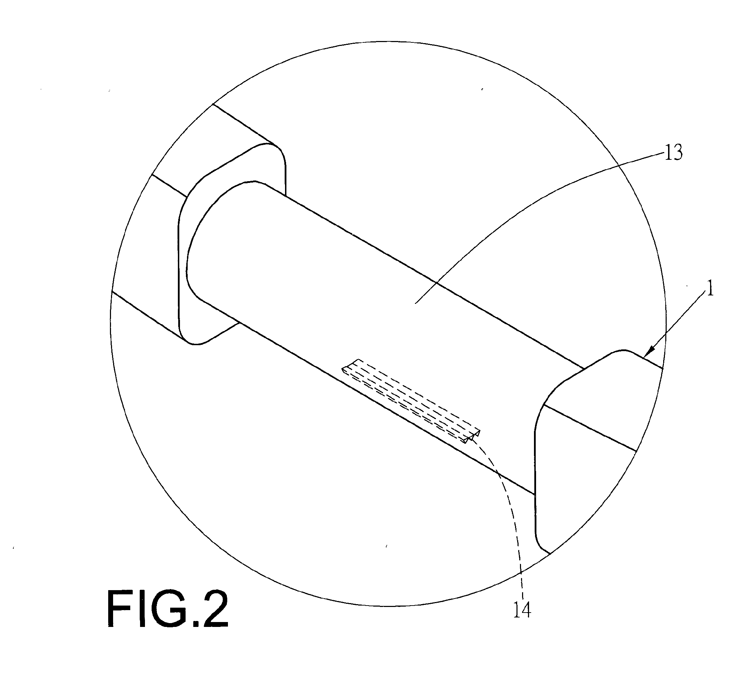

Referring to Figs. 1 and 2, the protection mask of the present invention comprises a frame 1 having two slits 10 defined in two ends thereof and a tongue 11 is located in each slit 10. Two temples 12 extend backward from the two ends of the frame 1. A connection rod 13 is located at the middle portion of the frame 1. The connection rod 13 of the frame 1 has at least one tooth 14 defined therein.

A connector 2 is connected to the connection rod 13 of the frame 1 and has a C-clip 20 on one end thereof. The C-clip 20 has a notch 21 in the middle portion thereof. A protrusion 22 extends from the connector 2 and has an annular groove 23 defined in the outside thereof.

A nose pad 3 is connected to the connection rod 13 of the frame 1 and has a snapping portion 30 which is engaged with the notch 21 of the connector 2. The snapping portion 30 has a snapping slot 31, and multiple engaging slots 32 are defined in the inside of the snapping slot 31 of the nose pad 3. The at least one tooth 14 is engaged with one of the engaging slots 32 of the snapping slot 31 of the nose pad 3.

A protection member 4 has an insertion slot 40 defined through the middle portion thereof as so to be connected in front of the frame 1. The protrusion 22 of the connector 2 is engaged with the insertion slot 40. Two connection slots 41 are respectively defined in two ends of the protection member 4. The protection member 4 is a transparent plate or a translucent and colored plate which stops ultra-violet rays from penetrating through the protection member 4.

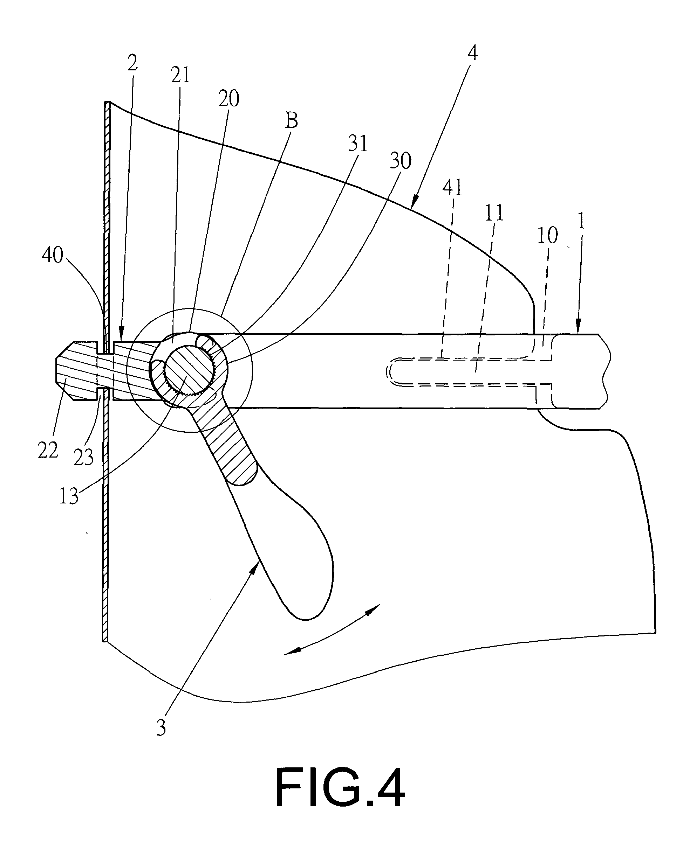

As shown in Figs. 1 to 5, when assembling the protection mask, the snapping slot 31 of the snapping portion 30 of the nose pad 3 is snapped to the connection rod 13 to engage the at least one tooth 14 with one of the engaging slots 32 of the snapping slot 31 of the nose pad 3. The C-clip 20 of the connector 2 is then connected to the connection rod 13, and the snapping portion 30 of the nose pad 3 is engaged with the notch 21 of the connector 2. The protection member 4 is then connected to the connector 2 by inserting the protrusion 22 of the connector 2 through the insertion slot 40 of the protection member 4. The periphery of the insertion slot 40 is engaged with the annular groove 23 of the protrusion 22 as shown in Fig. 4. The protection member 4 is then connected to the frame 1 by engaging the tongues 11 of the frame 1 with the connection slots 41 of the protection member 4. By this way, the protection member 4 is firmly connected to the frame 1.

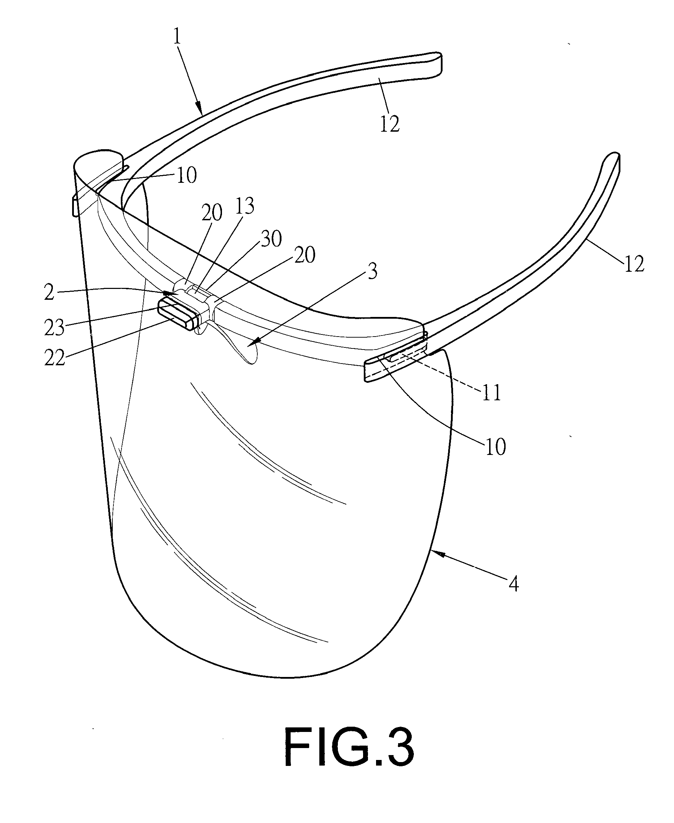

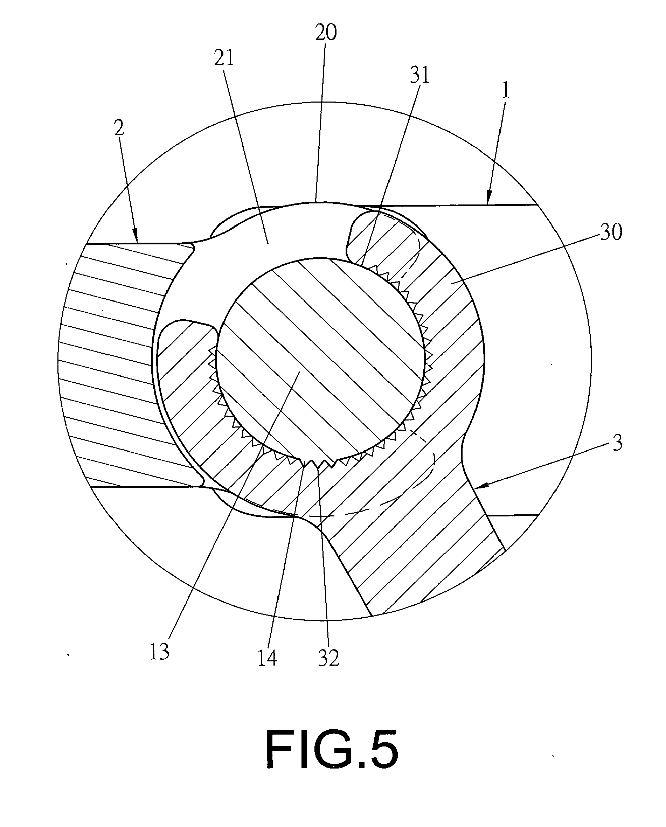

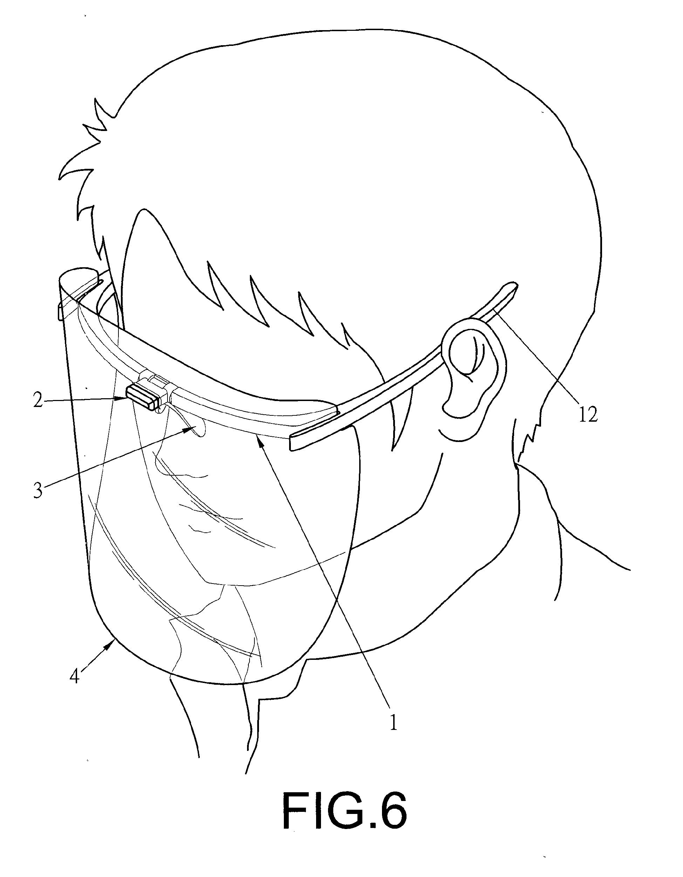

As shown in Figs. 3 to 7, when in use, the two temples 12 are located above the wearer's ears and the nose pad 3 is put on the wearer's nose as shown in Fig. 6. The protection member 4 can cover up the wearer's face. If the protection member 4 is a translucent and colored plate, it stops ultra-violet rays from penetrating through the protection member 4 and dust is keep out from the wearer's face. If the protection member 4 is a transparent plate, it protects the wear from being accessed by splash oil, smoke, patients' secretions and saliva. The nose pad 3 is rotated relative to the connection rod 13 as shown in Fig. 4 so that the height and angle of the nose pad 3 can be adjusted to meet requirements of different wearers. The at least one tooth 14 of the connection rod 13 of the frame 1 is engaged with one of the engaging slots 32 of the snapping slot 31 of the nose pad 3 as shown in Fig. 5 so position the nose pad 3 on the wearer's nose, so that the protection mask does not slip and shake.

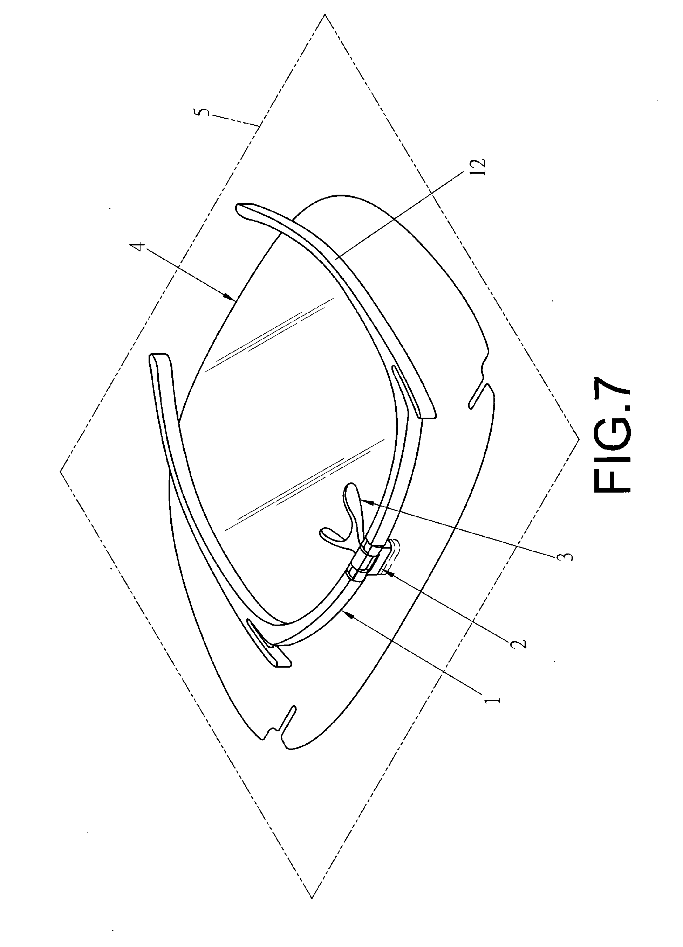

When folding the protection member 4, as shown in Fig. 7, the tow ends of the protection member 4 is removed from the two slits 10 of the frame 1, the protection member 4 and the connector 2 are rotated relative to the connection rod 13, so that the protection member 4 is folded downward to reduce the size of the protection mask. The folded protection mask is smaller in size so as to be easily packed, transported and carried. When the protection member 4 needs to be replaced, the protection member 4 can be removed from the protrusion 22 of the connector 2 to be separated from the frame 1. A new protection member 4 is then installed to the frame 1.

While we have shown and described the embodiment in accordance with the present invention, it should be clear to those skilled in the art that further embodiments may be made without departing from the scope of the present invention.

## Claims

1. A protection mask in characterized that:
a frame 1 having two slits 10 defined in two ends thereof, each slit 10 having a tongue 11 located therein, two temples 12 extending backward from the two ends of the frame 1, a connection rod 13 located at a middle portion of the frame 1;
a connector 2 connected to the connection rod 13 of the frame 1 and having a C-clip 20 on one end thereof, the C-clip 20 having a notch 21 in a middle portion thereof, a protrusion 22 extending from the connector 2 and having a groove 23 defined in an outside thereof;
a nose pad 3 connected to the connection rod 13 of the frame 1 and having a snapping portion 30 which is engaged with the notch 21 of the connector 2, the snapping portion 30 having a snapping slot 31, and
a protection member 4 deposited in front of the frame 1, having an insertion slot 40 defined through a middle portion thereof, the protrusion 22 of the connector 2 engaged with the insertion slot 40, two connection slots 41 respectively defined in two ends of the protection member 4, the two tongues 11 respectively inside of the slits 10 engaged with the two connection slots 41.

2. The protection mask as claimed in claim 1, wherein the connection rod 13 of the frame 1 has at least one tooth 14, multiple engaging slots 32 are defined in an inside of the snapping slot 31 of the nose pad 3, the at least one tooth 14 of the connection rod 13 of the frame 1 engaged with one of the engaging slots 32 of the snapping slot 31 of the nose pad 3.

3. The protection mask as claimed in claim 1, wherein the protection member 4 is a transparent plate.

4. The protection mask as claimed in claim 1, wherein the protection member 4 is a translucent and colored plate which stops ultra-violet rays from penetrating through the protection member 4.
